# EUROPÄISCHE PATENTSCHRIFT

(11) **EP 1 453 821 B1**
(45) Veröffentlichungstag und Bekanntmachung des Hinweises auf die Patenterteilung: **20.01.2010**
(21) Anmeldenummer: 02785395.1
(22) Anmeldetag: 19.11.2002
(51) Int. Cl.: C07D 315/00

(54) **VERFAHREN ZUR HERSTELLUNG VON ALKYLKETENDIMEREN**
METHOD FOR PRODUCING ALKYL KETENE DIMERS
PROCEDE DE PRODUCTION DE DIMERES D'ALKYLCETENE

(30) Priorität: 30.11.2001 DE 10158661
(43) Veröffentlichungstag der Anmeldung: 08.09.2004
(73) Patentinhaber: BASF SE, 67056 Ludwigshafen (DE)
(72) Erfinder: ETTL, Roland, 67454 Hassloch (DE); WINTER, Manfred, 67596 Dittelsheim-Hessloch (DE); FREUND, Torsten, 67117 Limburgerhof (DE); KESSLER, Thomas, 67105 Schifferstadt (DE); GRIMM, Günther, B-1180 Brüssel (BE)
(86) Internationale Anmeldenummer: PCT/EP2002/012932
(87) Internationale Veröffentlichungsnummer: WO 2003/045936

(56) Entgegenhaltungen:
- EP-A- 0 550 107
- EP-A- 0 612 739
- DE-A- 3 434 212

## Beschreibung

Die Erfindung betrifft ein Verfahren zur Herstellung von Alkylketendimeren durch Umsetzung von Carbonsäurechloriden mit tertiären Aminen in Abwesenheiten von Lösemitteln unter intensivem Mischen und Isolierung der Alkylketendimere aus dem Reaktionsgemisch.

Aus der EP-A-0550107 ist ein Verfahren zur Herstellung von Alkylketendimeren durch Umsetzung von Carbonsäurechloriden mit Triethylamin in Abwesenheit von Lösemitteln bekannt. Das Carbonsäurechlorid wird unter intensivem Mischen in das Triethylamin mit einer Geschwindigkeit von nicht mehr als 3 mol pro Stunde pro mol Triethylamin eingespeist, wobei das Vermischen, die Einspeisungsgeschwindigkeit und der Wärmeaustausch so geregelt werden, daß die Viskosität der Mischung bei weniger als 250 mPas gehalten wird, gemessen bei 60°C (Schergeschwindigkeit größer als 100 set⁻¹). Das überschüssige Amin wird anschließend aus der Reaktionsmischung durch Behandlung mit verdünnter Salzsäure extrahiert. Die Umsetzung erfolgt in dem Temperaturbereich von 50 bis 100, bevorzugt 55 bis 65°C.

Aus der EP-A-0612739 ist ebenfalls ein Verfahren zur Herstellung von Alkylketendimeren durch Umsetzung von Fettsäurehalogeniden mit tertiären Aminen bekannt. Bei diesem Verfahren setzt man mindestens 1,15 mol tertiäres Amin pro mol Fettsäurehalogenid ein und führt die Reaktion in Gegenwart von nicht mehr als 10 Gew.-%, bezogen auf die Menge an Fettsäurehalogenide, eines zusätzlichen Lösemittels durch. Das Alkylketendimer wird durch Strippen des tertiären Amins und nachfolgende Extraktion mit verdünnten Säuren erhalten.

Aus der EP-A-0684940 ist ebenfalls ein Verfahren zur Herstellung von Alkylketendimeren aus Carbonsäurehalogeniden und tertiären Aminen bekannt. Die Reaktion erfolgt diskontinuierlich, wobei man die Umsetzung in Gegenwart eines Ausgangsreaktionsgemisches vornimmt, das Alkylketendimer und bereits hergestellte Kristalle eines Hydrohalogenids von tertiärem Amin enthält und in Gegenwart von nicht mehr als 10 Gew.-% eines Lösemittels, bezogen auf Fettsäurehalogenide, arbeitet.

Aus der EP-A-0741121 ist die Herstellung von Alkylketendimeren durch Umsetzung von Fettsäurehalogeniden mit tertiären Aminen in inerten organischen Lösemitteln bekannt. Um das Reaktionsgemisch aufzuarbeiten, destilliert man zunächst das inerte organische Lösemittel zum größten Teil ab, fügt dann Wasser bzw. Wasserdampf zu und destilliert weiter. Die so erhältlichen Alkylketendimere weisen nur geringe Mengen an restlichem Lösemittel auf und haben in der Regel Alkylketendimer-Gehalte von ca. 90 Gew.-%. Selbst ein geringer Anteil an restlichem Lösemittel in Alkylketendimeren ist für viele Anwendungen nachteilig.

Der vorliegenden Erfindung liegt die Aufgabe zugrunde, lösemittelfreie Alkylketendimere in einer hohen Raum-ZeitAusbeute und mit einem Gehalt an Alkylketendimer von mindestens 89 Gew.-% herzustellen.

Die Aufgabe wird erfindungsgemäß gelöst mit einem Verfahren zur Herstellung von Alkylketendimeren durch Umsetzung von Carbonsäurechloriden mit tertiären Aminen in Abwesenheit von Lösemitteln unter intensivem Mischen und Isolierung der Alkylketendimere aus dem Reaktionsgemisch, wenn man Carbonsäurechloride und tertiäre Amine im Molverhältnis 1 : 1 bis 1 : 1,6 bei Temperaturen von 65 bis 150°C und einer Verweilzeit von 1 bis 30 Minuten umsetzt, die Viskosität des Reaktionsgemisches 300mPas bis 100 Pas (bestimmt in einem Physica Rotationsviskosimeter) beträgt, man Carbonsäurechloride und tertiäre Amine jeweils getrennt voneinander dosiert, die Reaktionspartner dann miteinander mischt, und das Reaktionsgemisch mit Hilfe von Knetern oder Extrudern gefördert wird.

Während das bei der Reaktion gebildete Alkylketendimere unter den Reaktionsbedingungen flüssig ist, sind die bei der Umsetzung entstehenden Salze im Reaktionsgemisch praktisch nicht löslich und führen zu einer starken Erhöhung der Viskosität der Mischung. So betragen die Viskositäten der Reaktionsmischung bei einer Temperatur von 60°C erfindungsgemäß 300 mPas bis zu 100 Pas (gemessen in einem Physica Rotationsviskometer). Die Viskosität des Reaktionsgemisches beträgt vorzugsweise 500 bis 8 000 mPas.

Alkylketendimere können beispielsweise mit Hilfe der allgemeinen Formel 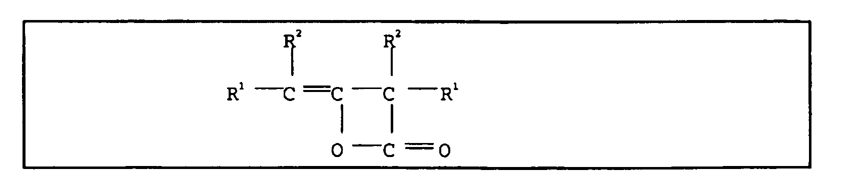 charakterisiert werden, in der
- R¹: C₄- bis C₃₀-Alkyl oder C₄- bis C₃₀-Alkenyl und
- R²: Wasserstoff oder C₁- bis C₈-Alkyl
bedeuten, wobei die Alkylreste R¹ und, sofern R² Alkyl bedeutet, jeweils linear oder verzweigt sein können. Die bei der Umsetzung verwendeten Carbonsäurechloride haben z.B. die allgemeine Formel 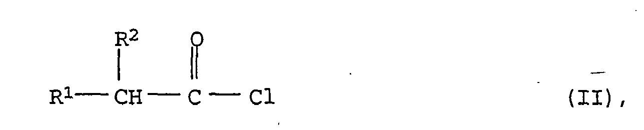 in der R¹ und R² die in Formel I angegebenen Bedeutungen haben. Die Carbonsäurechloride II haben vorzugsweise 14 bis 22 C-Atome. Man kann auch Gemische verschiedener Carbonsäurechloride einsetzen. Von technischer Bedeutung sind z.B. Gemische, die durch Chlorierung von natürlich vorkommenden Fettsäuren erhältlich sind, wie Säurechloride auf Basis von Fettsäuren, die aus Kokosöl, Tallöl, Rizinusöl, Olivenöl, Rindertalg oder Palmkernfett gewonnen werden. Typische Beispiele für Säurechloride sind Myristinsäurechlorid, Palmitinsäurechlorid, Stearinsäurechlorid, Behensäurechlorid, Ölsäurechlorid und Isostearinsäurechlorid.

Als tertiäre Amine kann man Monoamine oder Diamine mit mindestens einer Methylgruppe einsetzen. Bevorzugt sind Monoamine der allgemeinen Formel 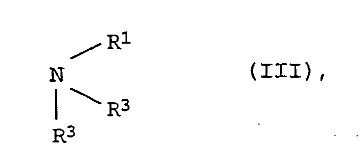 in der R¹, R², R³ unabhängig voneinander C₁- bis C₆-Alkyl, C₂- bis C₆-Alkenyl oder C₅- bis C₆-Cycloalkyl sind oder R¹ und R² über eine Alkylenkette aus bis zu 6 C-Atomen, vorzugsweise 4 oder 5 C-Atomen verbunden sind. Beispiele für Monoamine sind Dimethylcyclohexylamin, Diethylmethylamin, Dimethylethylamin, Trimethylamin, Triethylamin, Dimethylisopropylamin, N-Methylpiperidin, N-Methylpyrrolidin, Dimethylbutylamin und Dimethyl-2-ethylhexylamin. Beispiele für Diamine sind N,N,N',N'-Tetramethylpropandiamin, N,N,N',N'-Tetramethylhexandiamin und N,N-Dimethyl-N',N'-diethylpropandiamin. Mischungen aus Monoaminen und Diaminen sind möglich. Bevorzugt eingesetzte tertiäre Amine sind Dimethylcyclohexylamin, Dimethylisopropylamin, Dimethylethylamin und N-Methylpiperidin.

Carbonsäurechloride und tertiäre Amine werden im Molverhältnis 1 : 1 bis 1 : 1,6, vorzugsweise im Molverhältnis 1 : 1 bis 1 : 1,25 und besonders bevorzugt im Molverhältnis 1 : 1,02 bis 1 : 1,10 zur Reaktion gebracht. Die Umsetzung wird bei Temperaturen von 65 bis 150, vorzugsweise bei Temperaturen von 70 bis 110°C und insbesondere bei Temperaturen von 80 bis 110°C durchgeführt. Die Reaktionspartner werden beispielsweise in statischen oder dynamischen Mischern intensiv gemischt, beispielsweise in Pumpen, Extrudern, Knetern oder Düsen. Erfindungsgemäß werden Carbonsäurechloride und tertiäre Amine jeweils getrennt voneinander dosiert, die Reaktionspartner dann miteinander gemischt, und das Reaktionsgemisch mit Hilfe von Knetern oder Extrudern gefördert.

Carbonsäurechloride und tertiäre Amine können beispielsweise auch in einer Mehrstoffdüse versprüht und gemischt werden. Die Umsetzung von Carbonsäurechloriden und tertiären Aminen verläuft sehr schnell und ist innerhalb von Minuten abgeschlossen. Beim Versprühen erhält man feinverteilte Produktströme von tertiären Aminen und Carbonsäurechloriden. Der mittlere Teilchendurchmesser der versprühten Reaktionsteilnehmer beträgt beispielsweise 1 ∝m bis 1000 ∝m, vorzugsweise 10 ∝m bis 100 ∝m. Mit Hilfe geeigneter Kühlaggregate wird die Reaktionstemperatur in dem oben angegebenen Bereich gehalten. Man kann beispielsweise Reaktoren verwenden, die für eine gute Durchmischung der Reaktionsteilnehmer sorgen und die eine große Wärmeaustauschfläche haben, z.B. Planetwalzenextruder. Eine weitere Möglichkeit, die Reaktionswärme aus der Reaktionszone zu entfernen, besteht in einer Siedekühlung. Hierbei verdampft das tert. Amin als leicht flüchtigere Komponente und wird nach Kondensation wieder in den Prozess eingeschleust. Die Umsetzung kann bei Normaldruck, unter vermindertem Druck oder auch unter erhöhtem Druck durchgeführt werden, z.B. in dem Druckbereich von 50 mbar abs. bis 100 bar abs., vorzugsweise 50 mbar abs. bis 10 bar abs.

Bei einer kontinuierlichen Arbeitsweise in einem Kneter kann man beispielsweise in dem Bereich von 50 mbar abs. bis 10 bar abs. und unter Anwendung von Siedekühlung arbeiten.

Die Umsetzung der Carbonsäurechloride mit den tertiären Aminen wird vorzugsweise kontinuierlich durchgeführt. Geeignete Vorrichtungen hierfür sind, kontinuierlich arbeitende Kneter die über eine Mischdüse und eine Fördereinrichtung verfügen. Die Reaktoren müssen außerdem über geeignete Wärmetauscher verfügen. Die Verweilzeit in der Reaktionszone beträgt beispielsweise 1 bis 30 min, vorzugsweise 1 bis 15 min.

Die Reaktion der tertiären Amine mit den Carbonsäurechloriden geschieht erfindungsgemäß durch voneinander getrennte kontinuierliche Zuführung der Reaktionskomponenten in kontinuierlich betriebene Extruder, z.B. in einem Zweischneckenextruder oder in einem Planetwalzenextruder, bei Temperaturen von 70 bis 120°C, vorzugsweise bei 90 bis 110°C. Besonders bevorzugt werden hierfür als Aminkomponente Dimethylisopropylamin, Dimethylethylamin, Dimethylcyclohexylamin, N-Methylmorpholin und Triethylamin eingesetzt.

Nach Beendigung der Umsetzung wird das Reaktionsgemisch in üblicher Weise aufgearbeitet, z.B. mit Hilfe physikalischer Methoden wie Zentrifugieren oder durch Lösen der Ammoniumsalze in Wasser oder in verdünnten Säuren. Dies erfolgt beispielsweise durch Extraktion der bei der Reaktion gebildeten Ammoniumsalze mit verdünnten Säuren, z.B. mit verdünnter Schwefelsäure (Konzentration ca. 2 bis 20 Ges.-%). Hierbei wird überschüssiges Amin als Ammoniumsalz zusammen mit den bei der Umsetzung gebildeten Ammoniumsalzen in der wässrigen Phase gelöst und abgetrennt. Zweckmäßigerweise geht man dabei so vor, daß man die Reaktionsmaische in einen mit einem Rührer versehenen Kessel einträgt, in dem man verdünnte Schwefelsäure vorlegt, die eine Temperatur von 65 bis 90°C, vorzugsweise von 70 bis 80°C hat. In einer bevorzugten Variante der Aufarbeitung der Reaktionsmaische bedient man sich eines dynamischen Mischers, in den die Maische senkrecht auf die rotierende Achse gefördert wird. Nach der Phasentrennung wird das abgetrennte Alkylketendimere vorzugsweise einer weiteren Extraktion mit Wasser in einem Mixer-Settler-Apparatur unterworfen.

Es verbleiben Alkylketendimere mit einem Diketengehalt von mindestens 89 Gew.-%. In den meisten Fällen beträgt der Diketengehalt mindestens 90 Gew.-%.

Die nach dem erfindungsgemäßen Verfahren erhältlichen Diketene werden als Hydrophobierungsmittel verwendet. Die wichtigste Anwendung dieser Verbindungen ist die Masseleimung von Papier. Hierzu setzt man dem Papierstoff vor der Entwässerung mit Hilfe von Schutzkolloiden in Wasser dispergierte Alkylketendimere zu. Die üblicherweise angewendeten Mengen betragen 0,05 bis 0,20 Gew.-%, bezogen auf trockenen Papierstoff.

Der in den Beispielen angegebene Lactongehalt ist der Gehalt an Alkylketendimer der Formel I in Gew.-%. Die angegebene Verweilzeit ist eine mittlere Verweilzeit, die mit geeigneten Methoden (z.B. Konz. eines Farbstoffs) gemessen wurde.

### Beispiel 1 (nicht erfindungsgemäß)

Die Reaktion wird in einem Rohrreaktor durchgeführt, der aus einem doppelwandigem Rohr mit einer Gesamtlänge von 100 cm und einem Innendurchmesser von 5 cm besteht. Der Rohrreaktor ist mit einem wandgängigen Intensivmischer ausgerüstet. Das Reaktorvolumen beträgt 122 ml. Am Reaktoreingang befinden sich zwei Einlaßstutzen. In der Mitte des Reaktors ist ein weiterer Stutzen für einen zusätzlichen Eduktaustrag oder eine Probenahme vorhanden. Der Reaktor enthält über die Länge verteilt drei Temperaturmeßstellen und am Ende einen Austragsstutzen. Der Intensivmischer ist so gestaltet, daß an der Stelle der Dosierung von tertiärem Amin und Carbonsäurechlorid eine intensive Mischung der beiden Reaktionspartner erfolgt und im weiteren Verlauf des Reaktors neben der Vermischung eine Förderung des Reaktionsgemisches zum Reaktorausgang gegeben ist. Die Temperatur des Reaktors wird über einen Heiz-/Kühlkreislauf gesteuert. Der Reaktionsaustrag wird entweder diskontinuierlich in einer Rührapparatur oder bevorzugt kontinuierlich aufgearbeitet (konventionell durch Säure, wässrige Extraktion). Bei der diskontinuierlichen Aufarbeitung wird eine Probe des Reaktionsaustrags in verdünnte Schwefelsäure bei 65°C eingerührt. Nach 30 minütigem Stehen tritt eine Phasentrennung ein. die wässrige Phase wird abgelassen. Die organische Phase wird erneut mit Wasser versetzt, 15 min bei 65°C gerührt. Nach der Phasentrennung wird die organische Phase abgetrennt und IR-spektroskopisch untersucht.

Bei der kontinuierlichen Aufarbeitung wird der gesamte Reaktionsaustrag direkt in eine zweistufige Mixer-Settler-Apparatur geführt und bei einer Temperatur von 65 bis 70°C mit Wasser, welches mit Säuren (z.B. H₂SO₄ oder HCl) angesäuert ist, das Ammoniumsalze extrahiert.

Die Reaktion wird gestartet, indem man gleichzeitig Stearinsäurechlorid und Dimethylcyclohexylamin über die Eingangsstutzen in den Reaktor mit Hilfe zweier Pumpen dosiert. Mit Hilfe der Dosiergeschwindigkeit kann die Verweilzeit des Reaktionsgemisches im Reaktor eingestellt werden. In einer Standardeinstellung werden 453 g (1,5 mol) Stearinsäurechlorid pro Stunde und 209 g (1,65 mol) Dimethylcyclohexylamin pro Stunde über die getrennten Einlaßstutzen in den oberen Teil des Reaktors dosiert. Die Gehäusetemperatur beträgt 60°C. Durch die freiwerdende Reaktionswärme steigt die Innentemperatur des Reaktionsgemisches auf 68 bis 71°C an (Meßstelle befindet sich in unmittelbarer Nachbarschaft zur Dosierstelle) und pegelt sich auf diesem Niveau ein. Nach einer Verweilzeit von ca. 10 min tritt am unteren Ende des Reaktors das Produkt aus. Das Temperaturniveau über den gesamten Reaktor liegt am Ausgang des Reaktors bei 66°C und bei 71°C an der Dosierstelle. Nach ca. 2 h Dauerbetrieb wird eine Probe des Reaktionsaustrags entnommen und diskontinuierlich aufgearbeitet, indem man das Reaktionsgemisch mit verdünnter Schwefelsäure 15 Minuten bei einer Temperatur von 65°C rührt. Nach 30 minütigem Stehen tritt eine Phasentrennung ein. Die organische Phase wird erneut mit Wasser extrahiert und die Phasentrennung wird abgewartet. Die wässrige Phase wird abgelassen und die organische Phase IR-spektroskopisch untersucht. Man ermittelt einen Lactongehalt von 90,8 %.

### Beispiel 2 (nicht erfindungsgemäß)

Man verwendet den in Beispiel 1 beschriebenen Rohrreaktor und führt ihm stündlich über die Eingangsstutzen jeweils 906 g 3 mol Stearinsäurechlorid und 406 g (3,2 mol) Dimethylcyclohexylamin zu. Die Dosierung der Produkte erfolgt über 2 Waagen. Die Gehäusetemperatur des Reaktors beträgt 60°C. Infolge der Reaktionswärme steigt die Temperatur des Reaktionsgemisches auf 75 bis 79°C an und fällt zum Reaktorausgang auf 70 bis 72°C ab. Nach einer Verweilzeit von ca. 5 Minuten wird das Reaktionsprodukt über den Ausgangsstutzen des Reaktors ausgetragen. In einer daran angeschlossenen Mixer-Settler-Apparatur erfolgt die wässrige Extraktion des gebildeten Aminhydrochlorids. Das Stearyldiketen trennt sich als obere Phase ab und wird kontinuierlich entfernt. Die IR-spektroskopische Analyse ergab einen Lactongehalt von 92,7 % bei einer Probenahme nach 3,5 h Dauerbetrieb.

### Beispiel 3 (erfindungsgemäß)

Man verwendet als Reaktor einen Kneter, der mit temperierbaren, innenliegenden Wellen ausgestattet ist. Das Innenvolumen des Reaktors beträgt 0,47 1. Die Wellen und das Gehäuse des Kneters sind getrennt über externe Heiz/Kühlkreisläufe temperierbar. Die Dosierung der beiden Reaktionspartner erfolgt über einen gemeinsamen Einlaßstutzen, wobei das Säurechlorid und das Amin über eine Mischdüse vorgemischt und unmittelbar in den Reaktor dosiert werden. Die Temperatur des Reaktionsgemisches wird über mit der Reaktorwand abschließende Thermofühler gemessen. Der Reaktor wird auf 60°C vorgewärmt, die innenliegenden Wellen auf 52°C. Man dosiert jeweils pro Stunde 825 g Dimethylcyclohexylamin und 1750 g Talgfettsäurechlorid über die Mischdüse in den Reaktor. Die mittlere Verweilzeit des Reaktionsgemisches im Reaktor beträgt 9,5 Minuten. Das Reaktionsgemisch hat bei Eintritt in den Reaktor eine Temperatur von 78 bis 79°C und am Ausgang des Reaktors eine Temperatur von 71 bis 73°C. Das Reaktionsprodukt wird wie im Beispiel 1 beschrieben aufgearbeitet. Es hat einen Lactongehalt von 90,9 %.

### Beispiel 4 (erfindungsgemäß)

Man verwendet den in Beispiel 3 beschriebenen Kneter, stellt jedoch die Gehäusetemperatur und die Temperatur der Innenwellen auf 75°C ein. Der Reaktor wird kontinuierlich betrieben, indem man pro Stunde jeweils 1167 g N-Methylpiperidin und 3185 g Talgfettsäurechlorid in den Reaktor pumpt. Die Temperatur des Reaktionsgemisches steigt auf 94 bis 97°C an. Sie liegt am Ausgang des Reaktors bei 88°C. Das Reaktionsgemisch wird wie in Beispiel 1 beschrieben aufgearbeitet. Das Alkylketendimer hat einen Lactongehalt von 90,1 %.

### Beispiel 5 (erfindungsgemäß)

Man verwendet den in Beispiel 3 beschriebenen Reaktor, stellt jedoch die Gehäusetemperatur auf 70°C und die Temperatur der Innenwellen auf 55°C ein. Der Reaktor wird kontinuierlich betrieben, indem man ihm stündlich 1167 g N-Methylpiperidin und gleichzeitig 3185 g Talgfettsäurechlorid kontinuierlich zuführt. Die Innentemperatur des Reaktionsgemisches beträgt am Reaktoreingang 84 bis 87°C und am Reaktorausgang ca. 79°C, die mittlere Verweilzeit ca. 5,5 min. Das Reaktionsgemisch wird wie im Beispiel 1 beschrieben aufgearbeitet. Das Alkylketendimer hat einen Lactongehalt von 92,5 %.

### Beispiel 6 (nicht erfindungsgemäß)

Der Reaktor besteht aus einer Mischdüse, einem doppelwandigem Rohr (Innendurchmesser 9 mm), welches als Kreis ausgebildet ist und einer Pumpe. Die Mischdüse ist so angebracht, daß die beiden Edukte in nächster Nähe zur Pumpe gemischt und dann zur Pumpe weitergefördert werden. Ein weiteres, doppelwandiges, gerades Rohr (Innendurchmesser 9 mm) ist als Abzweigung an dem ersten kreisförmigem Rohr in einer Art und Weise angebracht, daß die beiden Reaktanden zuerst die Pumpe und den kreisförmigen Rohrreaktor durchlaufen müssen bevor sie in das gerade Rohr eintreten können. Die im kreisförmigen Rohrreaktor installierte Pumpe hat eine regelbare Förderleistung von 2 kg/h bis 20 kg/h.

Der gesamte Reaktor ist mit drei Temperaturmeßstellen sowie zwei Meßstellen zur Druckmessung ausgestattet. Die Temperatur wird über zwei unabhängig voneinander regelbare Heizkreise gesteuert. Das ermittelte gesamte Reaktorvolumen beträgt 102 ml, davon 65 ml im kreisförmigen Reaktor und 37 ml geraden Rohr. Die Temperatur des Heizkreises für den kreisförmigen Rohrreaktor wird auf 60°C eingestellt, die Temperatur für den geraden Rohrreaktor auf 66°C.

Die Reaktion wird gestartet, indem gleichzeitig 186 g Dimethylisopropylamin/h und 584 g Talgfettsäurechlorid/h über die Mischdüse in den kreisförmig ausgebildeten Rohrreaktor dosiert werden. Die Innentemperatur in diesem Reaktorteil steigt rasch auf 76 bis 80°C an und pegelt sich auf diesem Wert ein. Nach einer Verweilzeit von ca. 7 min tritt am Reaktorende das Produkt aus und kann in üblicher Art und Weise nach pH-Einstellung durch wässrige Extraktion aufgearbeitet werden. Die Temperatur in zweiten Reaktorteil pegelt sich auf 71 bis 72°C ein; im Reaktoraustrag werden 70°C gemessen. Nach Aufarbeitung des Austrags wird IR-spektroskopisch ein Lactongehalt von 92,1 % gemessen.

### Beispiel 7 (nicht erfindungsgemäß)

Man verwendet den in Beispiel 6 beschriebenen Reaktor. Die Temperatur des Heizkreises für den kreisförmigen Rohrreaktor wird auf 60°C eingestellt, die Temperatur für den geraden Rohrreaktor auf 95°C.

Die Reaktion wird gestartet, indem gleichzeitig 186 g Dimethylisopropylamin/h und 584 g Talgfettsäurechlorid/h über die Mischdüse in den kreisförmig ausgebildeten Rohrreaktor dosiert werden. Die Innentemperatur in diesem Reaktorteil steigt rasch auf 76 bis 80°C an und pegelt sich auf diesem Wert ein. Nach ca. 7 min Verweilzeit tritt am Reaktorende das Produkt aus und kann in üblicher Art und Weise nach pH-Einstellung durch wässrige Extraktion aufgearbeitet werden. Die Temperatur im zweiten Reaktorteil pegelt sich auf 87 bis 90°C ein. Am Reaktorausgang wird eine Temperatur von 88°C gemessen. Nach Aufarbeitung des Austrags wird in der abgetrennten oberen Phase IR-spektroskopisch ein Lactongehalt von 90,4 % gemessen.

### Beispiel 8 (erfindungsgemäß)

Ein Zweischneckenextruder mit einem freien Volumen von 1,2 dm³, wurde auf 90°C temperiert. In diesen Apparat wurden Talgfettsäurechlorid (TFSC1) und Dimethyl-iso-propylamin (DMiPA) in den in Tabelle 1 angegebenen Massenströmen mittels zweier Pumpen eindosiert. Durch die exotherme Reaktion stellten sich Innentemperaturen zwischen 95 und 106°C ein. Der Reaktionsaustrag wurde kontinuierlich aufgearbeitet, indem die Reaktionsmaische in einem dynamischen Mischer, der direkt an den Extruder angeschlossen war, mit dem 0,362fachen Massestrom 10,6 %iger Schwefelsäure vermischt wurde. Das feste Dimethylisopropylammonium-Hydrochlorid löste sich in der wässrigen Phase. Man erhielt zwei flüssige Phasen. Anschließend wurde die Schmelze des Ketendimeren von der sauren Lösung des Ammoniusalzes getrennt und zweimal in einer kontinuierlich arbeitenden Mixer-Settler Kaskade bei 75°C mit Wasser gewaschen. Die β-Lactongehalte der Ketendimeren sind in Tabelle 1 angegeben.

**Tabelle 1**

| Beispiel | TFSC1 | DMiPA | β-Lacton |
|---|---|---|---|
| | [kg/h] | [kg/h] | [%] |
| 8a | 12,60 | 4,32 | 93,1 |
| 8b | 15,75 | 5,40 | 90,6 |
| 8c | 18,90 | 6,48 | 90,6 |
| 8d | 22,06 | 7,56 | 91,3 |

### Beispiel 9 (erfindungsgemäß)

Ein Planetwalzenextruder mit einem freien Volumen von 1,7 dm³, bestehend aus zwei Schüssen, die mit 6 Mischerelementen bestückt waren, wurde mit einem externen Medium in den jeweiligen Schüssen temperiert. In diesen Apparat wurden Talgfettsäurechlorid (TFSC1) und Dimethyl-iso-propylamin (DMiPA) in den in Tabelle 2 angegebenem Verhältnis mittels zweier Pumpen eindosiert. Der Reaktionsaustrag wurde diskontinuierlich aufgearbeitet, indem die Reaktionsmaische in einem Rührbehälter mit der 0,362-fachen Masse 10,6 %iger Schwefelsäure vermischt wurde. Das feste Dimethylisopropylammonium-Hydrochlorid löste sich in der wässrigen Phase. Man erhielt zwei flüssige Phasen. Anschließend wurde die Schmelze des Ketendimeren von der sauren Lösung des Ammoniumsalzes getrennt und zweimal bei 75°C mit Wasser gewaschen. Die β-Lactongehalte der Ketendimeren sind in Tabelle 2 angegeben.

**Tabelle 2**

| Versuch | Verhältnis mol Amin/mol TFSC1 | Durchsatz kg/h | Temp °C Zyl 1 | Temp °C Zyl 2 | IR-Analytik Mittelwerte Lacton % |
|---|---|---|---|---|---|
| 9a | 1,122 | 39,0 | 60 | 80,0 | 92,2 |
| 9b | 1,127 | 51,5 | 70 | 72,5 | 93,3 |
| 9c | 1,121 | 42,4 | 80 | 65,0 | 92,9 |
| 9d | 1,121 | 25,5 | 80 | 80,0 | 92,8 |
| 9e | 1,171 | 32,2 | 90 | 87,5 | 91,1 |
| 9f | 1,122 | 42,6 | 100 | 80,0 | 89,3 |
| 9g | 1,0575 | 40,0 | 90 | 80 | 91,7 |
| 9h | 1,105 | 40,0 | 90 | 80 | 92,8 |
| 9i | 1,105 | 40,0 | 90 | 80 | 93,6 |
| 9k | 1,2 | 40,0 | 70 | 80 | 93,3 |

Beispiele 9a - 9f wurden mit Dimethylisopropylamin, die Beispiele 9g - 9k mit Dimethylethylamin durchgeführt.

### Beispiel 10 (nicht erfindungsgemäß)

Vergleich der Papierleimung eines Alkylketendimeren mit einem handelsüblichen Akylketendimeren

Das nach Beispiel 1 (a) hergestellte ALkylketendimere wurde bei 70°C geschmolzen und in eine Lösung aus 0,1 % Ligninsulfonat als Dispergiermittel, 2 % kationische Stärke und Wasser (ad 100 %) mittels eines Ultraturrax Rührers bei 80°C mit einem Gehalt an Ketendimeren von 6 % dispergiert. Die Rohemulsion wurde über einen Hochdruckhomogenisator (APV) bei 200 bar zweimal homogenisiert und auf 20°C abgekühlt. Die so erhaltene AKD-Dispersion wurde auf einer Versuchspapiermaschine in den in Tabelle 3 angegebenen Konzentrationen, die auf trockenen Papierstoff bezogen sind, zugesetzt. Der Papierstoff enthielt 30 Gew.-% Kiefernsulfat, 70 Gew.-% Birkensulfat und 20 Gew.-% CaCO₃ (Hydrocarb^{®} OG der Firma Omya). Die Hydrophobie der so erhaltenen Papiere eines Flächengewichtes von 80 g/m² wurde mittels des Cobb 60s Wertes und des HST-Wertes bestimmt.

Bestimmung des Leimungsgrades (HST-Wert) mit dem Hercules-Sizing-Tester:

Die Papierprobe wird in den Halter eingeklemmt; 10 ml der Prüftinte werden auf die Probe gegossen und die Messung wird gestartet. Wenn der gewählte Endpunkt des Reflexionsgrades erreicht wird, bleibt das Zeitzählwerk stehen. Die Zeit wird notiert, und die Penetrationszeit wird in Sekunden angegeben.

### Vergleichsbeispiel 1

Zum Vergleich wurde ebenfalls eine 6 % Alkylketendimer enthaltende wässrige Dispersion aus einem handelsüblichen Alkylketendimer (Basoplast® 20 konz, Lactongehalt 85 %) nach der oben beschriebenen Methode hergestellt und, wie oben beschrieben, als Leimungsmittel geprüft.

**Tabelle 3**

| Alkylketendimer hergestellt nach | Leimung bestimmt gemäß | Menge an Alkylketendimer, bezogen auf trockenen Papierstoff [Gew.-%] | | | | | |
|---|---|---|---|---|---|---|---|
| | | 0,06 | 0,072 | 0,084 | 0,096 | 0,108 | 0,12 |
| Beispiel 1c | Cobb 60s | 60 | 47 | 32 | 29 | 25 | 24 |
| Vergl.-Bsp. 1 | Cobb 60s | 72 | 67 | 44 | 30 | 26 | 25 |
| Beispiel 1c | HST | 9 | 12 | 40 | 71 | 126 | 163 |
| Vergl.-Bsp. 1 | HST | 2 | 6 | 37 | 62 | 118 | 124 |

### Vergleichsbeispiel 2

In einer doppelwandigen 2 1-Rührapparatur, ausgestattet mit einem wandgängigem Rührer, einem Thermoelement und einer Dosiervorrichtung, werden 580 g trockenes Toluol und 279 g (2,20 mol) getrocknetes Dimethylcyclohexylamin vorgelegt und auf 50°C erwärmt. Nach dem Erreichen der Innentemperatur von 50°C werden unter intensivem Rühren 584 g (2,00 mol) Talgfettsäurechlorid (C₁₆/C₁₈-Gemisch) aus einem Behälter, der auf einer Waage steht, mit Hilfe einer Dosierpumpe mit einer Rate von 234 g/h Talgfettsäurechlorid in den Reaktor gepumpt. Durch die freiwerdende Reaktionswärme steigt die Temperatur rasch auf 70°C an. Die Viskosität der Reaktionsmischung nimmt ebenfalls deutlich zu. Über einen externen Heiz-/Kühlkreislauf wird dafür gesorgt, daß die Temperatur nicht über 75°C steigt. Nach beendeter Dosierung von Stearinsäurechlorid wird die Mischung noch 45 min bei 65 bis 70°C nachgerührt. Anschließend wird die Reaktionsmischung langsam mit 20,6 g konz. Schwefelsäure versetzt, dann mit 400 ml Wasser und 15 min bei 65°C gerührt. Nach 30 min Phasentrennung wird die wässrige Phase abgelassen. Die organische Phase wird erneut mit 250 g Wasser gewaschen. Nach Abtrennung der wässrigen Phase wird das Lösungsmittel unter Vakuum abdestilliert.

Man erhält 507 g Alkylketendimer mit einem Lactongehalt von 85,3 % (IR-spektroskopische Analyse).

### Vergleichsbeispiel 3 (Vergleich nach EP-A-550 107)

In der im Vergleichsbeispiel 2 beschriebenen Rührapparatur werden 230 g (2,27 mol) Triethylamin vorgelegt und auf 50°C erwärmt. Dann werden 614 g (2,08 mol) Talgfettsäurechlorid im Verlauf von 60 min zudosiert. Die Temperatur des Heizkreislaufs wird so geregelt, daß die Innentemperatur nicht über 65°C steigt. Die dickflüssige Reaktionsmischung wird noch 15 min bei 65°C nachgerührt, dann mit 416 ml einer 10 %igen Salzsäure versetzt und 15 min bei 60°C gerührt. Dann wird der Rührer abgestellt. Nach 15 min wird die wässrige Phase abgelassen. Man gibt 100 g Wasser zu und rührt die Mischung 15 min bei 60°C. Der Rührer wird dann abgestellt und die wässrige Phase nach 30 min abgelassen. Die organische Phase hat einen Lactongehalt von ca. 85,4 % (IR-spektroskopische Analyse).

## Patentansprüche

1. Verfahren zur Herstellung von Alkyldiketenen durch Umsetzung von Carbonsäurechloriden mit tertiären Aminen in Abwesenheit von Lösemitteln unter intensivem Mischen und Isolierung der Alkyldiketene aus dem Reaktionsgemisch, **dadurch gekennzeichnet, daß** man Carbonsäurechloride und tertiäre Amine im Molverhältnis 1 : 1 bis 1 : 1,6 bei Temperaturen von 65 bis 150°C und einer Verweilzeit von 1 bis 30 Minuten umsetzt, die Viskosität des Reaktionsgemisches 300 mPas bis 100 Pas (bestimmt in einem Physica Rotationsviskosimeter) beträgt, man Carbonsäurechloride und tertiäre Amine jeweils getrennt voneinander dosiert, die Reaktionspartner dann miteinander mischt, und das Reaktionsgemisch mit Hilfe von Knetern oder Extrudern gefördert wird.

2. Verfahren nach Anspruch 1, **dadurch gekennzeichnet, daß** die Viskosität des Reaktionsgemisches 500 mPas bis 8 000 mPas (bestimmt bei 60°C in einem Physica Rotationsviskometer) beträgt.

3. Verfahren nach einem der Ansprüche 1 bis 2, **dadurch gekennzeichnet, daß** man die Umsetzung kontinuierlich durchführt.

4. Verfahren nach einem der Ansprüche 1 bis 3, **dadurch gekennzeichnet, daß** man die Umsetzung in einem Zweischneckenextruder oder in einem Planetwalzenextruder durchführt, wobei man Carbonsäurechloride und tertiäre Amine getrennt voneinander kontinuierlich in einen der genannten Extruder dosiert und das Reaktionsgemisch nach dem Durchlaufen des Extruders kontinuierlich austrägt.

5. Verfahren nach einem der Ansprüche 1 bis 3, **dadurch gekennzeichnet, daß** man die Umsetzung bei Temperaturen von 70 bis 120°C durchführt.

6. Verfahren nach einem der Ansprüche 1 bis 5, **dadurch gekennzeichnet, daß** die Verweilzeit des Reaktionsgemisches im Reaktor 1 bis 15 Minuten beträgt.

## Claims

1. A process for the preparation of alkyldiketenes by reacting acyl chlorides with tertiary amines in the absence of solvents with thorough mixing and isolation of the alkyldiketenes from the reaction mixture, wherein acyl chlorides and tertiary amines are reacted in a molar ratio of from 1 : 1 to 1 : 1.6 at from 65 to 150°C and with a residence time of from 1 to 30 minutes, the viscosity of the reaction mixture is from 300 mPa.s to 100 Pa.s (determined in a Physica rotational viscometer), acyl chlorides and tertiary amines are each metered in separately from one another, the reactants are then mixed with one another and the reaction mixture is conveyed with the aid of kneaders or extruders.

2. The process according to claim 1, wherein the viscosity of the reaction mixture is from 500 to 8 000 mPa.s (determined at 60°C in a Physica rotational viscometer).

3. The process according to any of claims 1 to 2, wherein the reaction is carried out continuously.

4. The process according to any of claims 1 to 3, wherein the reaction is carried out in a twinscrew extruder or in a planetary roller extruder, acyl chlorides and tertiary amines being metered separately from one another and continuously into one of said extruders and the reaction mixture being discharged continuously after passing through the extruder.

5. The process according to any of claims 1 to 3, wherein the reaction is carried out at from 70 to 120°C.

6. The process according to any of claims 1 to 5, wherein the residence time of the reaction mixture in the reactor is from 1 to 15 minutes.

## Revendications

1. Procédé pour la préparation d'alkyldicétènes par transformation de chlorures d'acides carboxyliques avec des amines tertiaires en l'absence de solvants par mélange intensif et isolement des alkyldicétènes du mélange réactionnel, **caractérisé en ce qu'**on transforme des chlorures d'acides carboxyliques et des amines tertiaires dans un rapport molaire de 1:1 à 1:1,6 à des températures de 65 à 150°C et un temps de séjour de 1 à 30 minutes, la viscosité du mélange réactionnel est de 300 mPa.s à 100 Pa.s (déterminée dans un viscosimètre de Physica), on dose les chlorures d'acides carboxyliques et les amines tertiaires à chaque fois séparément les uns des autres, on mélange ensuite les partenaires de réaction les uns avec les autres et on transporte le mélange réactionnel à l'aide de malaxeurs ou d'extrudeuses.

2. Procédé selon la revendication 1, **caractérisé en ce que** la viscosité du mélange réactionnel est de 500 mPa.s à 8000 mPa.s (déterminée à 60°C dans un viscosimètre Physica).

3. Procédé selon l'une quelconque des revendications 1 à 2, **caractérisé en ce qu'**on réalise la transformation en continu.

4. Procédé selon l'une quelconque des revendications 1 à 3, **caractérisé en ce qu'**on réalise la transformation dans une extrudeuse à double vis sans fin ou dans une extrudeuse à cylindres planétaire, où on dose les chlorures d'acides carboxyliques et les amines tertiaires en continu, séparément les uns des autres dans une des extrudeuses mentionnées et on évacue le mélange réactionnel en continu après le passage dans l'extrudeuse.

5. Procédé selon l'une quelconque des revendications 1 à 3, **caractérisé en ce qu'**on réalise la transformation à des températures de 70 à 120°C.

6. Procédé selon l'une quelconque des revendications 1 à 5, **caractérisé en ce que** le temps de séjour du mélange réactionnel dans le réacteur est de 1 à 15 minutes.
